# EUROPEAN PATENT SPECIFICATION

(11) **EP 0 920 410 B1**
(45) Date of publication and mention of the grant of the patent: **27.03.2002**
(21) Application number: 97939508.4
(22) Date of filing: 22.08.1997
(51) Int. Cl.: C07C 227/08, C07C 247/14, C07D 317/46, C07D 203/26, C07D 303/40, C07F 7/18, C07C 227/16

(54) **PREPARATION OF CYCLOHEXENE CARBOXYLATE DERIVATIVES**
VERFAHREN ZUR HERSTELLUNG VON CYCLOHEXEN-CARBOXYLATDERIVATE
PREPARATION DE DERIVES DE CYCLOHEXENE CARBOXYLATE

(30) Priority: 23.08.1996 US 24122 P; 23.08.1996 US 701942
(43) Date of publication of application: 09.06.1999
(73) Proprietor: GILEAD SCIENCES, INC., Foster City CA 94404 (US)
(72) Inventor: KENT, Kenneth, M., Sunnyvale, CA 94086 (US); KIM, Choung, U., San Carlos, CA 94070 (US); MCGEE, Lawrence, R., Pacifica, CA 94044 (US); MUNGER, John, D., Alviso, CA 95002 (US); PRISBE, Ernest, J., Los Altos, CA 94024 (US); POSTICH, Michael, J., San Mateo, CA 94403 (US); ROHLOFF, John, C., Mountain View, CA 94040 (US); KELLY, Daphne, E., San Francisco, CA 94122 (US); WILLIAMS, Matthew, A., Foster City, CA 94404 (US); ZHANG, Lijun, Foster City, CA 94404 (US)
(74) Representative: Kinzebach, Werner, Dr.
(86) International application number: US9714813
(87) International publication number: WO9807685

(56) References cited:
- WO-A-96/26933
- G. ULIBARRI ET AL: "Construction of the bicyclic core structure of the enediyne antibiotic esperamicin-A1 in either enantiomeric form from (-)-quinic acid" JOURNAL OF ORGANIC CHEMISTRY, vol. 60, no. 9, 1995, pages 2753-2761, XP002050553
- L. CHAHOUA ET AL : "Synthesis of (-)-shikimate and (-)-quinate 3-phosphates by differentiation of the hydroxyl functions of (-)-shikimic and (-)-quinic acids" JOURNAL OF ORGANIC CHEMISTRY, vol. 57, no. 21, 1992, pages 5798-5801, XP002050554
- S. FERNANDEZ ET AL: "New and efficient enantiospecific synthesis of (-)-methyl 5-epi-shikimate and methyl 5-epi-quinate from (-)-quinic acid" TETRAHEDRON LETTERS, vol. 38, no. 29, 21 July 1997, pages 5225-5228, XP002050555
- C. U. KIM ET AL : "influenza neuraminidase inhibitors possessing a novel hydrophobic interaction in the enzyme active site: design, synthesis and structural analysis of carboxylic sialic acid analogues with potent anti-influenza activity" JOURNAL OF THE AMERICAN CHEMICAL SOCIETY, vol. 119, no. 4, February 1997, pages 681-690, XP002050556

## Description

The present invention is directed to methods of preparing carbocyclic compounds and intermediates therefore.

### Brief Description of Related Art

United States Patent Application (having Attorney Docket No. 205.6) Serial Number 08/702,308, filed August 23,1996, which was a continuation-in-part application of United States Patent Application Serial Number 08/653,034, filed March 24,1996, which was a continuation-in-part application of United States Patent Application Serial Number 08/606,624, filed February 26, 1996, which was a continuation-in-part application of United States Patent Application Serial Number 08/580,567, filed December 29, 1995, which was a continuation-in-part application of United States Patent Application Serial Number 08/476,946, filed June 6, 1995, which was a continuation-in-part application of United States Patent Application Serial Number 08/395,245, filed February 27, 1995, all of which are incorporated herein by reference in their entirety, describe, inter alia, neuraminadase inhibitors and intermediates in the synthesis of neuraminidase inhibitor. The present invention provides processes useful in the preparation of these compositions.

### Objects of the Invention

Selected embodiments of the invention are directed to one or more of the following objects:

A principal object of the invention is to provide new synthetic methods and compositions.

An additional object of the invention is to provide new methods of preparing intermediates useful in the synthesis of neuraminidase inhibitors.

An additional object of the invention is to provide compounds useful as intermediates that are themselves useful in the synthesis of neuraminidase inhibitors.

An additional object of the invention is to provide compounds useful as neuraminidase inhibitors.

### Summary of the Invention

The first aspect of the present invention ist directed to a process for the preparation of a compound of the formula: 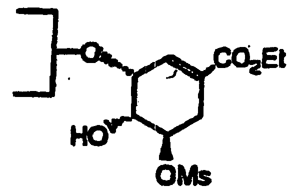 wherein Ms is mesyl;
which process comprises reaction of a compound of the formula: 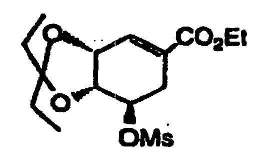 with a Lewis acid reagent.

The second aspect of the present invention is directed to a process for the preparation of a compound of the formula: 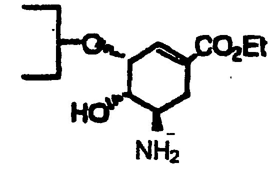 which process comprises reaction of a compound of the formula: 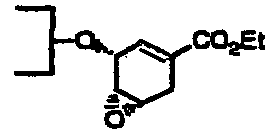 with an amine reagent.

The third aspect of the present invention is directed to compounds of the formulae

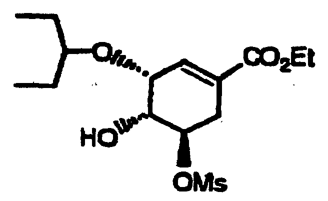

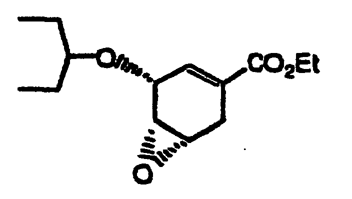

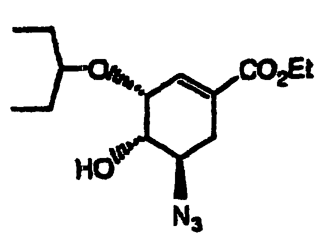

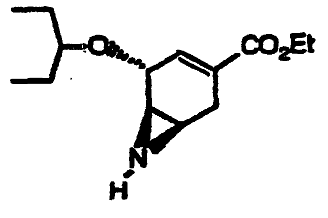

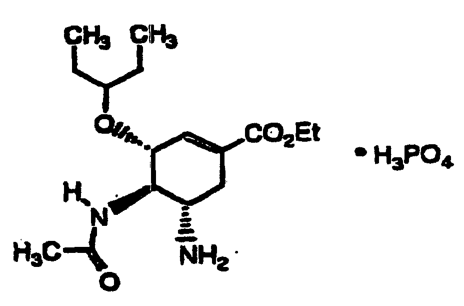

The fourth aspect of the present invention is directed to a process for preparing the compound of the formula 116 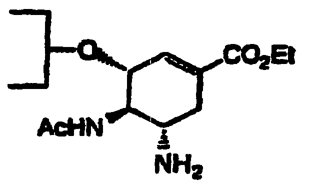 which comprises
a) converting the compound of formula 110 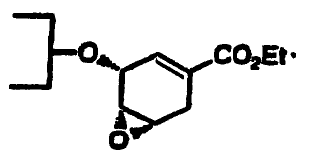 to the compound of formula 111: 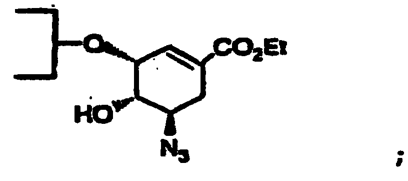
b) converting the compound of formula 111 to the compound of formula 113: 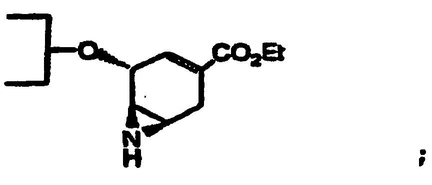
c) converting the compound of formula 113 to the compound of formula 114: 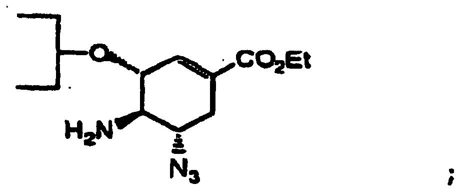
d) converting the compound of formula 114 to the compound of formula 115: 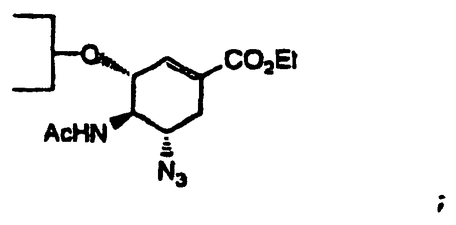 and
e) converting the compound of formula 115 to the compound of formula 116.

The fifth aspect of the present invention is directed to a process for preparing the compound of formula 116: 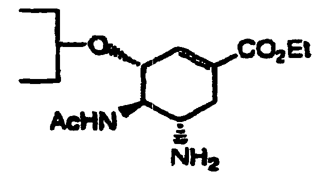 which comprises
a) converting the compound of formula 201 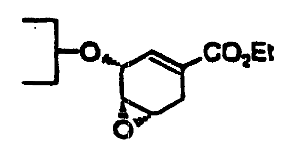 to the compound of formula 202 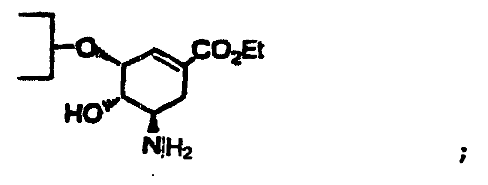
b) converting the compound of formula 202 to the compound of formula 203 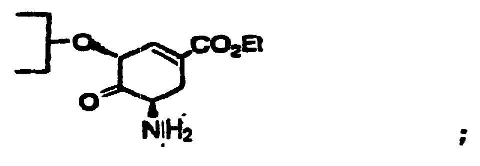
c) converting the compound of formula 203 to the compound of formula 204 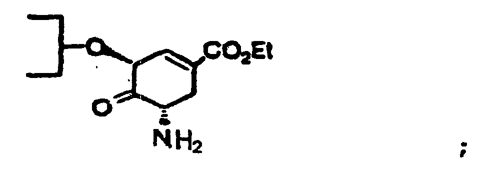
d) converting the compound of formula 204 to the compound of formula 205 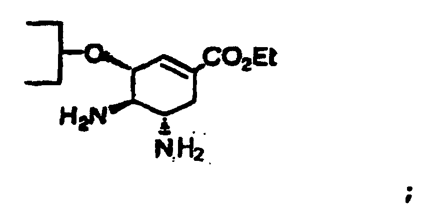 and
e) converting the compound of formula 205 to the compound of formula 116.

### Detailed Description

### General

The present invention is directed to methods of making the compounds described herein. Even though the compounds of the invention are prepared by any of the applicable techniques of organic synthesis, the present invention provides advantageous methods for accomplishing the preparations.

Many conventional techniques are well known in the art and will not be elaborated here. However, many of the known techniques are elaborated in "Compendium of Organic Synthetic Methods" (John Wiley & Sons, New York), Vol. 1, Ian T. Harrison and Shuyen Harrison, 1971; Vol. 2, Ian T. Harrison and Shuyen Harrison, 1974; Vol. 3, Louis S. Hegedus and Leroy Wade, 1977; Vol. 4, Leroy G. Wade, jr., 1980; Vol. 5, Leroy G. Wade, Jr., 1984; and Vol. 6, Michael B. Smith; as well as March, J., "Advanced Organic Chemistry, Third Edition", (John Wiley & Sons, New York, 1985), "Comprehensive Organic Synthesis. Selectivity, Strategy & Efficiency in Modern Organic Chemistry. In 9 Volumes", Barry M. Trost, Editor-in-Chief (Pergamon Press, New York, 1993 printing).

Generally, the reaction conditions such as temperature, reaction time, solvents, workup procedures, and the like, will be those common in the art for the particular reaction to be performed. The cited reference material, together with material cited therein, contains detailed descriptions of such conditions.

The terms "treated", "treating", "treatment", and the like, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two.

"Treating" indicates the reasonable and usual manner in which organic chemicals are allowed to react. Normal concentrations (0.01M to 10M, typically 0.1M to 1M), temperatures (-100°C to 250°C, typically -78°C to 150°C, more typically -78°C to 100°C, still more typically 0°C to 100°C), solvents (aprotic or protic), reaction times (typically 10 seconds to 10 days, more typically 1 min. to 10 hours, still more typically 10 min. to 6 hours), reaction vessels (typically glass, plastic, metal), pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic sysnthesis selects conditions and apparatus reasonably expected to successfully carry out the chemical reactions of the described processes based on the knowledge in the art.

Oxidation and reduction reactions are typically carried out at temperatures near room temperature (about 20°C), although for metal hydride reductions frequently the temperature is reduced to 0°C to -100°C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0°C to -100°C) are also common. Solvents can be either protic (common in equilibrating reactions) or aprotic (common in kinetically controlled reactions).

Standard synthetic techniques such as azeotropic removal of reaction by-products and use of anhydrous reaction conditions (e.g. inert gas environments) are common in the art and will be applied when applicable. Workup typically consists of quenching any unreacted reagents followed by partition between a water/organic layer system (extraction) and separating the layer containing the product. Each of the products of the following processes is optionally separated, isolated, and/or purified prior to its use in subsecquent processes.
The process embodiment according to the first aspect of the invention comprises reaction of a compound of the formula: 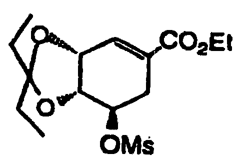 with a Lewis acid reagent.

Typically, compound 10 is reacted with a Lewis acid catalyst common in the art, such as BF₃•Et₂O, TiCl₃, TMSOTf, SmI₂(THF)₂, LiClO₄, Mg(ClO₄)₂, Ln(OTf)₃ (where Ln=Yb, Gd, Nd), Ti(Oi-Pr)₄, AlCl₃, AlBr₃, BeCl₂, CdCl₂, ZnCl₂, BF₃, BCl₃, BBr₃, GaCl₃, GaBr₃, TiCl₄, TiBr₄, ZrCl₄, SnCl₄, SnBr₄, SbCl₅, SbCl₃, BiCl₃, FeCl₃, UCl₄, ScCl₃, YCl₃, LaCl₃, CeCl₃, PrCl₃, NdCl₃, SmCl₃, EuCl₃, GdCl₃, TbCl₃, LuCl₃, DyCl₃, HoCl₃, ErCl₃, TmCl₃, YbCl₃, ZnI₂, Al(OPrⁱ)₃, Al(acac)₃, ZnBr₂, or SnCl₄. Optionally, compound 10 is also treated with a reducing reagent. Typical reducing reagents are of the form B(R³⁰)₃ such as BH₃. Optionally reducing reagents of the form B(R³⁰)₃ are complexed with common solvents such as diethylether and dimethylsulfide. A wide range of borane reducing reagents are known and will not be described in detail here. For example Brown, H.C. "Boranes in Organic Chemistry", (Cornell Univ. Press, Ithaca, N.Y., 1972) (Brown) provides a very large number of examples such as is found in Part Four, Selective Reductions, pages 209-251, Part Five, Hydroboration, pages 255-297, and Part Six, Organoboranes, pages 301-446.

In a typical embodiment, compound 10 is treated with a lewis acid in a nonprotic solvent. More typically, compound 10 is treated with a lewis acid and a reducing reagent in a nonprotic solvent.

In a typical embodiment, a solution of 10 in dichlorormethane is cooled and treated with borane-methyl sulfide complex and trimethylsilyl trifluoromethanesulfonate. 10% Aqueous sodium bicarbonate solution is slowly added. The mixture is warmed to ambient temperature and stirred. The organic layer is filtered and concentrated *in vacuo* to leave the desired compound. A detailed example of this embodiment is provided as Example 6 below.
The fourth aspect of the present invention includes the preparation of the compound of the formula:

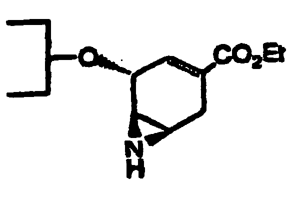

This process embodiment comprises reaction of a compound of the formula: 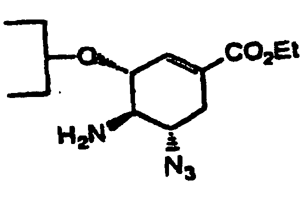 with a reducing reagent.

The azide of compound 30 is reduced to form compound 31.

Typically the process comprises treating compound 30 with a reducing agent to form compound 31. More typically the process comprises treating compound 30 with hydrogen gas and a catalyst (such as platinum on carbon or Lindlar's catalyst), or reducing reagents (typically a trisubstituted phosphine such as trialkyl or triaryl phosphine e.g. triphenylphosphine). More typically still, the process comprises treating compound 30 with triphenylphosphine and a base to form compound 31.

Typically, compound 30 is disolved in a suitable polar, aprotic solvent such as anhydrous acetonitrile. A solution of anhydrous triphenylphosphine in a suitable solvent such as anhydrous tetrahydrofuran or a mixture of solvents is added dropwise. The mixture is heated at reflux then concentrated *in vacuo* to leave compound 5. A detailed example of this embodiment is provided as Example 9 below.
The second aspect of the present invention is directed to a process for the preparation of the compound of the formula:

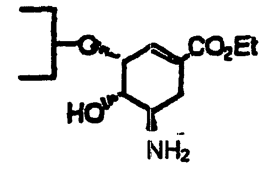

This process embodiment comprises reaction of a compound of the formula: 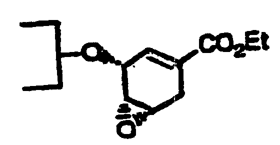 with an amine reagent. Typically, the amine reagent is of the formula HY¹ or a salt of HY¹, such as, by way of example, NH₃ (McManns, et al., "Bull Soc. Chim. France" 850 (1947)), HY¹ generally (Moussevon, M., et al., "Synth. Commun." 3:177 (1973)) or phthalimide (Gabriel, et al., "Ber." 20:2224 (1887) or Gibson, et al., "Angew. Chem. Int.", 7:919-930 (1968)).

The process comprises treating compound 40 with the amine reagent to produce compound 32. More typically, compound 40 is treated with the amine reagent in a suitable polar a protic solvent (e.g. CH₃CN, DMF or THF). Optionally compound 40 is treated with the amine reagent and a base. Typical details of this process embodiment can be found in March, "Advanced Organic Chemistry" 4th. ed., pp 425-427.
The fifth aspect of the present invention includes the preparation of the compound of the formula:

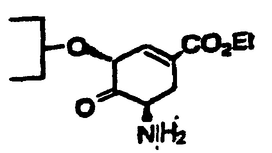

This process embodiment comprises reaction of the compound of the formula: 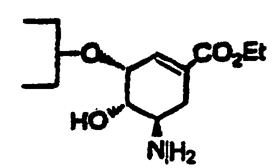 with an oxidizing reagent. A wide range of suitable oxidation reagents are common in the art and will not be detailed here. For example House, H.O. "Modern Synthetic Reactions, Second Edition", Chapter 5, pages 259-273, describes the selective oxidation of alcohols. Typical reagents include CrO₃, Na₂Cr₂O₇, KMnO₄, PDC and PCC. Typical details of this process embodiment can be found in Larock, "Comprehensive Organic Transformations", pp. 604-614; Corey et al., "Tetrahedron Lett." 31:2647-50 (1975); Ley et al., "Chem. Common" 1625 (1987); Sweon, et al., "J. Org. Chem." 43:2480-2 (1978); and Martin, et al., "J. Org, Chem." 48:4155-56 (1983). Solvents typically include inert polar solvents (e.g. CH₂Cl₂, toluene or CH₃CN).
The preparation of the compound of the formula: : 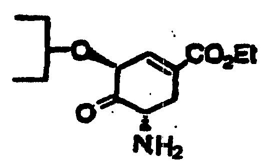 comprises reaction of compound 50 with a base. Typically the base is a hindered amine or hindered alkoxide or the salts of either. More typically the base is of the formula NaOR²⁵, KOR²⁵ or NR²⁵₃, more typically yet, DBN, DBU or diisopropyl ethyl amine.

The preparation of the compound of the formula: 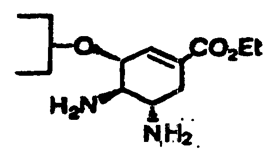 comprises reaction of compound 61 with a reductive amination reagent. Typical details of and references to this process embodiment can be found in Larock, *op*. *cit*., pp. 421-425. Another typical description (NaCNBH₃ method) is Borch, "J. Am. Chem. Soc." 93:2897-2904 (1971).

Schemes 1 and 2 depict embodiments of the invention. Detailed descriptions of the processes of Schemes 1 and 2 are provided in the Examples (below).

Additional individual process embodiments of the invention include any one or sequential combination of processes AA, AB, AC, AD, AE, AF, AG, AH, AI, AJ, or AK of Schemes 1 and 2. "Sequential combination" as used herein means more than one process wherein the individual processes are performed one after the other in the order shown. Isolation, separation, purification is optionally performed prior to any of the individual processes.

Additional individual process embodiments of the invention include any one or sequential combination of the processes of Example 1, Example 2, Example 3, Example 4, Example 5, Example 6, Example 7, Example 8, Example 9, Example 10, Example 11, Example 12 or Example 13.

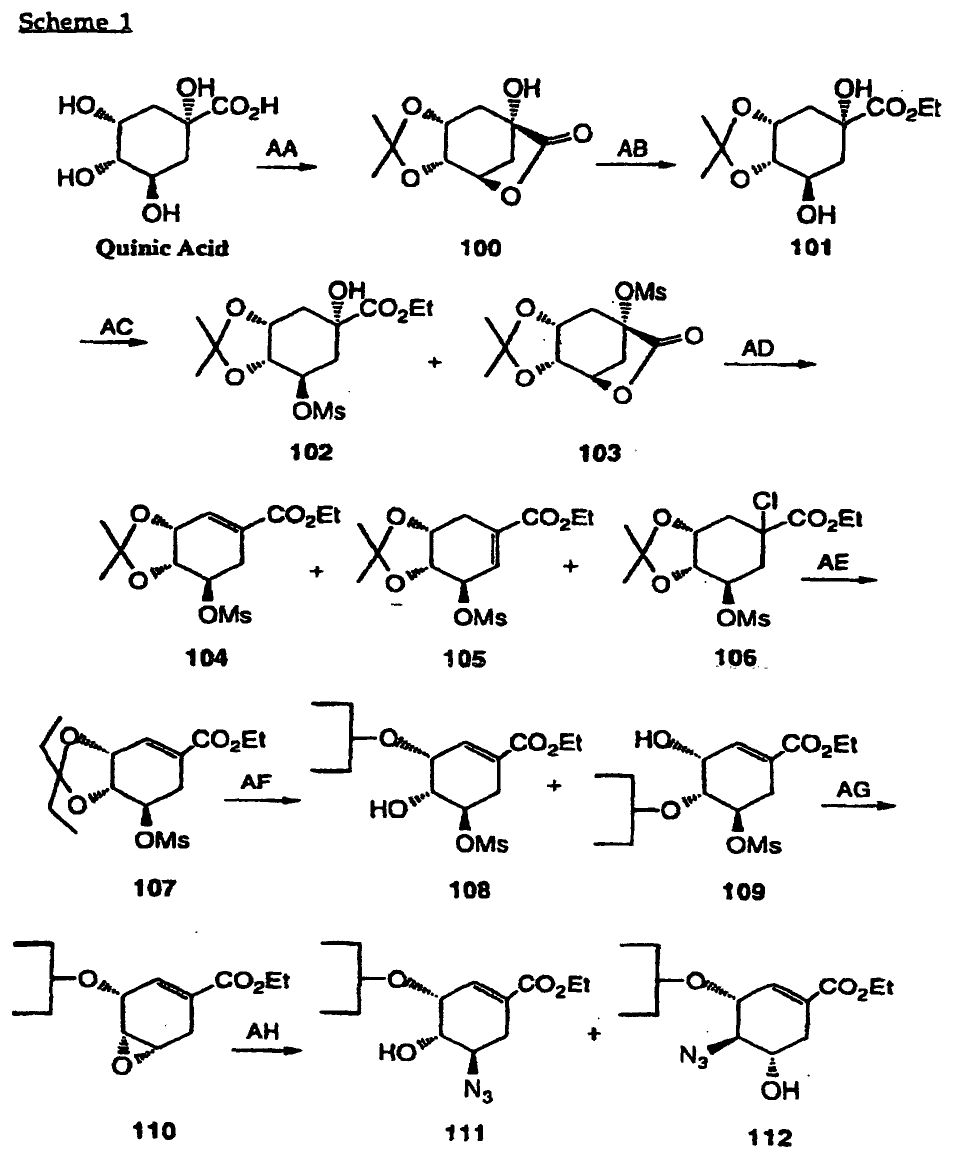

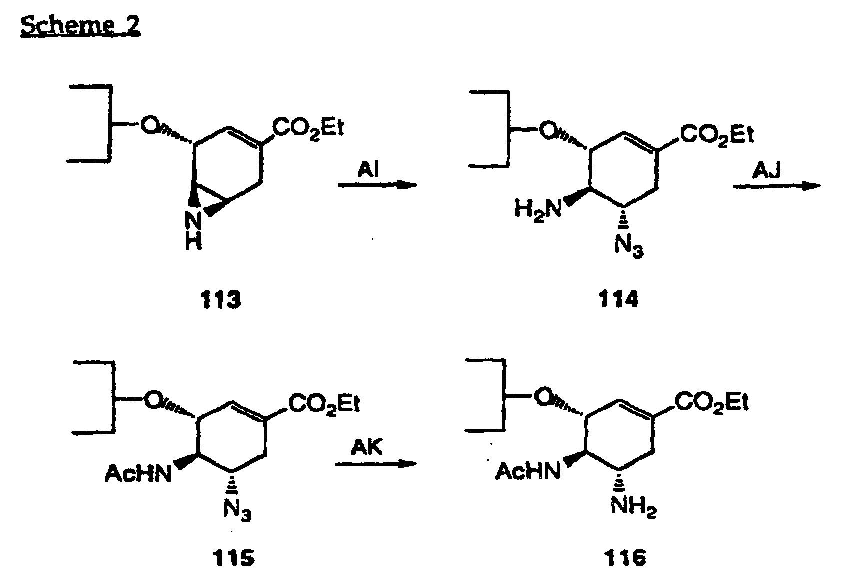

Scheme 3 depicts the synthesis of the neuraminidase inhibitor 206 (R = H₂) by use of alternative nitrogen nucleophiles (March, "Advanced Organic Chemistry" 4th. ed., pp 425-427) to open the epoxide 201. Oxidation of azidoalcohol 202 gives ketone 203 (Larock, "Comprehensive Organic Transformations", pp. 604-614) in which the β-axial NR group isomerizes to the α-equatorial configuration 204. Reductive amination of the ketone 204 (Larock, *op. cit*., pp. 421-425) gives the β-equatorial amine 205 which is acetylated to afford 206. Cleavage of the R moiety (Greene, "Protective Groups in Organic Synthesis", pp. 218-287) gives the neuramidase inhibitor 206 (R = H₂).

Additional individual process embodiments of the invention include any one or sequential combination of processes AL, AM, AN, AO, or AP of Scheme 3.

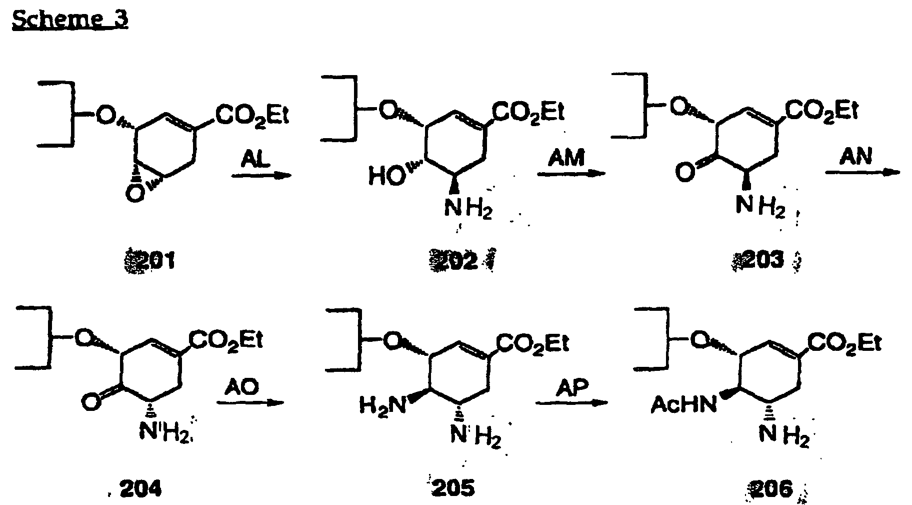

Modifications of each of the above schemes leads to various analogs of the specific exemplary materials produced above. The above cited citations describing suitable methods of organic synthesis are applicable to such modifications.

In each of the above exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example, size exclusion or ion exchange chromatography, high, medium, or low pressure liquid chromatography, small scale and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

### Stereoisomers

The compounds of the invention are enriched or resolved optical isomers at any or all asymmetric atoms. For example, the chiral centers apparent from the depictions are provided as the chiral isomers or racemic mixtures. Both racemic and diasteromeric mixtures, as well as the individual optical isomers isolated or synthesized, substantially free of their enantiomeric or diastereomeric partners, are all within the scope of the invention.

One or more of the following enumerated methods are used to prepare the enantiomerically enriched or pure isomers herein. The methods are listed in approximately their order of preference, i.e., one ordinarily should employ stereospecific synthesis from chiral precursors before chromatographic resolution before spontaneous crystallization.

Stereospecific synthesis is described in the examples. Methods of this type conveniently are used when the appropriate chiral starting material is available and reaction steps are chosen do not result in undesired racemization at chiral sites. One advantage of stereospecific synthesis is that it does not produce undesired enantiomers that must be removed from the final product, thereby lowering overall synthetic yield. In general, those skilled in the art would understand what starting materials and reaction conditions should be used to obtain the desired enantiomerically enriched or pure isomers by stereospecific synthesis. If an unexpected racemization occurs in a method thought to be stereospecific then one needs only to use one of the following separation methods to obtain the desired product.

If a suitable stereospecific synthesis cannot be empirically designed or determined with routine experimentation then those skilled in the art would turn to other methods. One method of general utility is chromotographic resolution of enantiomers on chiral chromatography resins. These resins are packed in columns, commonly called Pirkle columns, and are commercially available. The columns contain a chiral stationary phase. The racemate is placed in solution and loaded onto the column, and thereafter separated by HPLC. See for example, Proceedings Chromatographic Society - International Symposium on Chiral Separations, Sept. 3-4, 1987. Examples of chiral columns that could be used to screen for the optimal separation technique would include Diacel Chriacel OD, Regis Pirkle Covalent Dphenylglycine, Regis Pirkle Type 1A, Astec Cyclobond II, Astec Cyclobond III, Serva Chiral D-DL=Daltosil 100, Bakerbond DNBLeu, Sumipax OA-1000, Merck Cellulose Triacetate column, Astec Cyclobond I-Beta, or Regis Pirkle Covalent D-Naphthylalanine. Not all of these columns are likely to be effective with every racemic mixture. However, those skilled in the art understand that a certain amount of routine screening may be required to identify the most effective stationary phase. When using such columns it is desireable to employ embodiments of the compounds of this invention in which the charges are not neutralized, e.g., where acidic functionalities such as carboxyl are not esterified or amidated.

Another method entails converting the enantiomers in the mixture to diasteriomers with chiral auxiliaries and then separting the conjugates by ordinary column chromatography. This is a very suitable method, particularly when the embodiment contains free carboxyl, amino or hydroxyl that will form a salt or covalent bond to a chiral auxiliary. Chirally pure amino acids, organic adds or organosulfonic acids are all worthwhile exploring as chiral auxiliaries, all of which are well known in the art Salts with such auxiliaries can be formed, or they can be covalently (but reversibly) bonded to the functional group. For example, pure D or L amino acids can be used to amidate the carboxyl group of embodiments of this invention and then separated by chromatography.

Enzymatic resolution is another method of potential value. In such methods one prepares covalent derivatives of the enantiomers in the racemic mixture, generally lower alkyl esters (for example of carboxyl), and then exposes the derivative to enzymatic cleavage, generally hydrolysis. For this method to be successful an enzyme must be chosen that is capable of stereospecific cleavage, so it is frequently necessary to routinely screen several enzymes. If esters are to be cleaved, then one selects a group of esterases, phosphatases, and lipases and determines their activity on the derivative. Typical esterases are from liver, pancreas or other animal organs, and include porcine liver esterase.

If the enatiomeric mixture separates from solution or a melt as a conglomerate, i.e., a mixture of enantiomerically-pure crystals, then the crystals can be mechanically separated, thereby producing the enantiomerically enriched preparation. This method, however, is not practical for large scale preparations and is of no value for true racemic compounds.

Asymmetric synthesis is another technique for achieving enantiomeric enrichment. For example, a chiral protecting group is reacted with the group to be protected and the reaction mixture allowed to equilibrate. If the reaction is enantiomerically specific then the product will be enriched in that enantiomer.

Further guidance in the separation of enantiomeric mixtures can be found, by way of example and not limitation, in "Enantiomers, Racemates, and resolutions", Jean Jacques, Andre Collet, and Samuel H. Wilen (Krieger Publishing Company, Malabar, FL, 1991, ISBN 0-89464-618-4). In particular, Part 2, "Resolution of Enantiomer Mixture", pages 217-435; more particularly, section 4, "Resolution by Direct Crystallization", pages 217-251, section 5, "Formation and Separation of Diastereomers", pages 251-369, section 6, "Crystallization-Induced Asymmetric Transformations", pages 369-378, and section 7, "Experimental Aspects and Art of Resolutions", pages 378-435; still more particularly, section 5.1.4, "Resolution of Alcohols, Transformation of Alcohols into Salt-Forming Derivatives", pages 263-266, section 5.2.3, "Covalent Derivatives of Alcohols, Thiols, and Phenols", pages 332-335, section 5.1.1, "Resolution of Acids", pages 257-259, section 5.1.2, "Resolution of Bases", pages 259-260, section 5.1.3, "Resolution of Amino Acids", page 261-263, section 5.2.1, "Covalent Derivatives of Acids", page 329, section 5.2.2, "Covalent Derivatives of Amines", pages 330-331, section 5.2.4, "Covalent Derivatives of Aldehydes, Ketones, and Sulfoxides", pages 335-339, and section 5.2.7, "Chromatographic Behavior of Covalent Diastereomers", pages 348-354, are cited as examples of the skill of the art.

### Salts and Hydrates

The compositions of this invention optionally comprise salts of the compounds herein, for example, Na⁺, Li⁺, K⁺, Ca⁺⁺ and Mg⁺⁺. Such salts may include those derived by combination of appropriate cations such as alkali and alkaline earth metal ions or ammonium and quaternary amino ions with an acid anion moiety. Monovalent salts are preferred if a water soluble salt is desired.

Metal salts typically are prepared by reacting the metal hydroxide with a compound of this invention. Examples of metal salts which are prepared in this way are salts containing Li⁺, Na⁺, and K⁺. A less soluble metal salt can be precipitated from the solution of a more soluble salt by addition of the suitable metal compound.

In addition, salts may be formed from acid addition of certain organic and inorganic acids, e.g., HCl, HBr, H₂SO₄, H₃PO₄ or organic sulfonic acids, to basic centers, typically amines. Finally, it is to be understood that the compositions herein comprise compounds of the invention in their un-ionized, as well as zwitterionic form, and combinations with stoiochimetric amounts of water as in hydrates.

Also included within the scope of this invention are the salts of the parental compounds with one or more amino acids. Any of the amino acids described above are suitable, especially the naturally-occuring amino acids found as protein components, although the amino acid typically is one bearing a side chain with a basic or acidic group, e.g., lysine, arginine or glutamic acid, or a neutral group such as glycine, serine, threonine, alanine, isoleucine, or leucine.

### Additional Uses for the Compounds of This Invention

The compounds of the invention are polyfunctional. As such they represent a unique class of monomers for the synthesis of polymers. By way of example and not limitation, the polymers prepared from the compounds of this invention include polyamides, polyesters and mixed polyester-polyamides.

The present compounds are used as monomers to provide access to polymers having unique pendent functionalities. The compounds of this invention are useful as comonomers with monomers which do not fall within the scope of the invention. Polymers of the compounds of this invention will have utility as cation exchange agents (polyesters or polyamides) in the preparation of molecular sieves (polyamides), textiles, fibers, films, formed articles and the like. Polymers are prepared by any conventional method, for example, by cross-linking an -OH or -NH₂ group of the compounds of the invention with a diacid comonomer. The preparation of these polymers from the compounds of the invention is conventional per se.

The compounds of the invention are also useful as a unique class of polyfunctional surfactants. Particularly when R⁴ or R² do not contain hydrophilic substituents and are, for example, alkyl, the compounds have the properties of bi-functional surfactants. As such they have useful surfactant, surface coating, emulsion modifying, rheology modifying and surface wetting properties.

As polyfunctional compounds with defined geometry and carrying simultaneously polar and non-polar moieties, the compounds of the invention are useful as a unique class of phase transfer agents. By way of example and not limitation, the compounds of the invention are useful in phase transfer catalysis and liquid/liquid ion extraction (LIX).

The compounds of the invention optionally contain asymmetric carbon atoms. As such, they are a unique class of chiral auxiliaries for use in the synthesis or resolution of other optically active materials. For example, a racemic mixture of carboxylic acids can be resolved into its component enantiomers by: 1) forming a mixture of diastereomeric esters or amides with a compound of the invention containing an -OH or -NH₂ group; 2) separating the diastereomers; and 3) hydrolyzing the ester structure. Further, such a method can be used to resolve the compounds of the invention themselves if optically active acids are used instead of racemic starting materials.

The compounds of this invention are useful as linkers or spacers in preparing affinity absorption matrices, immobilized enzymes for process control, or immunoassay reagents. The compounds herein contain a multiplicity of functional groups that are suitable as sites for cross-linking desired substances. For example, it is conventional to link affinity reagents such as hormones, peptides, antibodies, drugs, and the like to insoluble substrates. These insolublized reagents are employed in known fashion to absorb binding partners for the affinity reagents from manufactured preparations, diagnostic samples and other impure mixtures. Similarly, immobilized enzymes are used to perform catalytic conversions with facile recovery of enzyme. Bifunctional compounds are commonly used to link analytes to detectable groups in preparing diagnostic reagents.

Many functional groups in the compounds of this invention are suitable for use in cross-linking. For example, -OH and -NH₂ groups. Suitable protection of reactive groups will be used where necessary while assembling the cross-linked reagent to prevent polymerization of the bifunctional compound of this invention. In general, the compounds here are used by linking them through hydroxyl or amino groups to carboxylic or phosphonic acid groups of the first linked partner, then covalently bonding to the other binding partner through another -OH or-NH₂ group. For example a first binding partner such as a steroid hormone is reacted to form an amide bond with the -NH₂ group of a compound of this invention and then this conjugate is cross-linked through a hydroxyl to cyanogen bromide activated Sepaharose, whereby immobilized steroid is obtained. Other chemistries for conjugation are well known. See for example Maggio, "Enzyme-Immunoassay" (CRC, 1988, pp 71-135) and references cited therein.

The following examples are put forth so as to provide those of ordinary skill in the art with a complete disclosure and description of how to make the compounds and compositions of the invention and are not intended to limit the scope of what the inventors regard as their invention. Efforts have been made to insure accuracy with respect to numbers used (e.g., amounts, temperatures, etc.), but some experimental errors and deviations should be taken into account. Unless indicated otherwise, parts are parts by weight, temperature is in degrees Centigrade, and pressure is at or near atmospheric.

### Examples

### Example 1

**Lactone 100:** A solution of quinic acid (20 kg, 104 mol; [α]_{D}-43.7° (c = 1.12, water); "Merck Index 11th ed"., 8071: [α]_{D} -42° to -44° (water)), 2,2-dimethoxypropane (38.0 kg, 365 mol) and *p*-toluenesulfonic acid monohydrate (0.200 kg, 1.05 mol) in acetone (80 kg) was heated at reflux for two hours. The reaction was quenched by addition of 21% sodium ethoxide in ethanol (0.340 kg, 1.05 mol) and most of the solvent was distilled *in vacuo*. The residue was partitioned between ethyl acetate (108 kg) and water (30 kg). The aqueous layer was back-extracted with ethyl acetate (13 kg) and the combined organic layers were washed with 5% aqueous sodium bicarbonate (14 kg). Most of the ethyl acetate was distilled *in vacuo* to leave a pale yellow solid residue of 100 which was used directly in the next step.

### Example 2

**Hydroxy ester 101:** A solution of the crude lactone 100 (from 104 mol. (-)-quinic acid) in absolute ethanol (70 kg) was treated with 20% sodium ethoxide in ethanol (0.340kg, 1.05 mol). After two hours at room temperature, acetic add (0.072 kg, 1.2 mol) was added and the solvent was distilled *in vacuo*. Ethyl acetate (36 kg) was added and the distillation continued to near dryness. The tan solid residue composed of a ca. 5:1 mixture of **101:100** was dissolved in ethyl acetate (9 kg) at reflux and hexane (9 kg) was added. Upon cooling, a white crystalline solid formed which was isolated by filtration to afford a ca. 6.5:1 mixture of **101:100** (19.0 kg, 70% yield).

### Example 3

**Mesyl ester 102:** A solution of a ca. 6.5:1 mixture (18.7 kg, ca. 72 mol) of hydroxy ester **101** and lactone **100** in dichloromethane (77 kg) was cooled to 0-10°C and treated with methanesulfonyl chloride (8.23 kg, 71.8 mol), followed by slow addition of triethylamine (10.1 kg, 100 mol). An additional portion of methanesulfonyl chloride (0.84 kg, 7.3 mol) was added. After one hour, water (10 kg) and 3% hydrochloric acid (11 kg) were added. The layers were separated and the organic layer was washed with water (9 kg), then distilled *in vacuo* to leave a semi-solid residue composed of a ca. 6.5:1 mixture of mesyl ester **102** and mesyl lactone **103**. The residue was dissolved in ethyl acetate (11 kg) and cooled to -10° to-20°C for two hours. Mesyl lactone **103** crystallized and was separated by filtration and washed with cold ethyl acetate (11 kg). The filtrate was concentrated to afford mesyl ester **102** as an orange resin (20.5 kg, 84.3% yield).

### Example 4

**Mesyl acetonide 104:** A solution of mesyl ester **102** (10.3 kg, 30.4 mol) and pyridine (10.4 kg, 183 mol) in dichloromethane (63 kg) was cooled to -20° to -30°C and treated portionwise with sulfuryl chloride (6.22 kg, 46 mol). After the exothermic reaction subsided, the resulting slurry was quenched with ethanol (2.4 kg), warmed to 0°C, and washed successively with 16% sulfuric acid (35 kg), water (15 kg) and 5% aqueous sodium bicarbonate (1 kg). The organic layer containing a ca. 4:1:1 mixture of **104:105:106** was concentrated *in vacuo* and ethyl acetate (14 kg) was added. The allylic mesylate **105** was selectively removed by treatment of the ethyl acetate solution with pyrrolidine (2.27 kg, 31.9 mol) and tetrakis(triphenylphosphine)palladium(0) (0.0704 kg, 0.061mol) at ambient temperature for five hours, followed by washing with 16% sulfuric acid (48 kg). The organic layer was filtered through a pad of silica gel (11 kg) and eluted with ethyl acetate (42 kg). The filtrate was concentrated *in vacuo* to leave a thick orange oil composed of a ca. 4:1 mixture of **104:106**. The residue was dissolved in ethyl acetate (5.3 kg) at reflux and hexane (5.3 kg) was added. Upon cooling, mesyl acetonide **104** crystallized and was separated by filtration and washed with 14% ethyl acetate in hexane (2.1 kg). After drying *in vacuo*, **104** was obtained as pale yellow needles (4.28 kg, 43.4% yield), mp 102-3°C.

### Example 5

**Pentyl ketal 107:** A solution of acetonide **104** (8.9 kg, 27.8 mol), 3-pentanone (24 kg, 279 mol) and 70% perchloric acid (0.056 kg, 0.39 mol) was stirred for 18 hours. The volatiles were distilled *in vacuo* at ambient temperature and fresh 3-pentanone (30 kg, 348 mol) was added gradually as the distillation progressed. The reaction mixture was filtered, toluene (18 kg) was added, and the resulting solution was washed successively with 6% aqueous sodium bicarbonate (19 kg), water (18 kg) and brine (24 kg). The organic layer was concentrated *in vacuo* and toluene (28 kg) was added gradually as the distillation progressed. When no more distilled, the residual orange oil was composed of pentyl ketal **107** (9.7 kg, 100% yield) and toluene (ca. 2 kg).

### Example 6

**Pentyl ether 108:** A solution of ketal **107** (8.6 kg, 25 mol) in dichloromethane (90 kg) was cooled to -30° to -20°C and treated with borane-methyl sulfide complex (2.1 kg, 27.5 mol) and trimethylsilyl trifluoromethanesulfonate (7.2 kg, 32.5 mol). After one hour, 10% aqueous sodium bicarbonate solution (40 kg) was slowly added. The mixture was warmed to ambient temperature and stirred for 12 hours. The organic layer was filtered and concentrated *in vacuo* to leave a ca. 8:1 mixture of **108:109** as a gray waxy solid (7.8 kg, 90% yield).

### Example 7

**Epoxide 110:** A ca. 8:1 mixture of isomeric pentyl ethers **108:109** (7.8 kg, 22.3 mol) in ethanol (26 kg) was treated with a solution of potassium hydrogen carbonate (3.52 kg, 35 mol) in water (22 kg). After heating at 55°-65°C for two hours, the solution was cooled and twice extracted with hexanes (31 kg, then 22 kg). Unreacted **109** remained in the aqueous ethanol layer. The combined hexane extracts were filtered and concentrated *in vacuo* to leave epoxide **110** as a flocculent white crystalline solid (3.8 kg, 60% yield), mp=54.6°C.

### Example 8

**Hydroxy azide 111:** A mixture of epoxide **110** (548 g, 2.0 mol), sodium azide (156 g, 2.4 mol) and ammonium chloride (128.4 g, 2.4 mol) in water (0.265 L) and ethanol (1.065 L) was heated at 70°-75°C for eight hours. Aqueous sodium bicarbonate (0.42 L of 8% solution) was added and the ethanol was distilled *in vacuo*. The aqueous residue was extracted with ethyl acetate (1 L) and the extract was washed with water (0.5 L). The water wash was back-extracted with ethyl acetate (0.5 L). The combined organic extracts were washed with brine (0.5 L), dried over anhydrous sodium sulfate, filtered and concentrated *in vacuo* to leave a ca. 10:1 mixture of isomeric hydroxy azides **111:112** (608 g, 102% yield) as a dark brown oil.

### Example 9

**Aziridine 113:** A ca. 10:1 mixture of hydroxy azides **111:112** (608 g, 2.0 mol) was three times co-evaporated *in vacuo* from anhydrous acetonitrile (3 x 0.3 L) and then dissolved in anhydrous acetonitrile (1 L). A solution of anhydrous triphenylphosphine (483 g, 1.84 mol) in anhydrous tetrahydrofuran (0.1 L) and anhydrous acetonitrile (0.92 L) was added dropwise over two hours. The mixture was heated at reflux for six hours then concentrated *in vacuo* to leave a golden paste composed of aziridine 113, triphenylphosphine oxide and traces of triphenylphosphine. The paste was triturated with diethyl ether (035 L). Most of the insoluble triphenylphosphine oxide was removed by filtration and washed with diethyl ether (1.5 L). The filtrate was concentrated *in vacuo* to leave a dark brown oil which was dissolved in 20% aqueous methanol and extracted three times with hexanes (3 x 1 L) to remove triphenylphosphine. The hexane extracts were back-extracted with 20% aqueous methanol (0.5 L) and the combined aqueous methanol layers were concentrated *in vacuo*. The residue was twice co-evaporated *in vacuo* from anhydrous acetonitrile (2 x 0.5 L) to leave a dark brown oil composed of aziridene **113** (490 g, 96.8 % yield) and triphenylphosphine oxide (ca. 108 g) which was used directly in the next step.

### Example 10

**Acetamido azide 115:** A mixture of aziridine **113** (490 g, 1.93 mol) and triphenylphosphine oxide (ca. 108 g), sodium azide (151 g, 2.33 mol) and ammonium chloride (125 g, 2.33 mol) in dimethylformamide (1.3 L) was heated at 80°-85°C for five hours. Sodium bicarbonate (32.8 g, 0.39 mol) and water (0.66 L) were added. The amino azide **114** was isolated from the reaction mixture by six extractions with hexanes (6 x 1 L). The combined hexane extracts were concentrated *in vacuo* to ca. 4.5 L total volume and dichloromethane (1.04 L) was added. Aqueous sodium bicarbonate (4.2 L of 8% solution, 3.88 mol) was added, followed by acetic anhydride (198 g, 1.94 mol). After stirring for one hour at ambient temperature, the aqueous layer was discarded. The organic phases were concentrated *in vacuo* to 1.74 kg total weight and dissolved with ethyl acetate (0.209 L) at reflux. Upon cooling, acetamido azide **115** crystallized and was isolated by filtration. After washing with cold 15% ethyl acetate in hexane (1 L) and drying *in vacuo* at ambient temperature, pure **115** was obtained as off-white crystals (361 g, 55% yield), mp 126-132°C.

### Example 11

**Acetamido amine 116:** A mixture of azide **115** (549 g, 1.62 mol) and Lindlar catalyst (50 g) in abs. ethanol (3.25 L) was stirred for eighteen hours while hydrogen (1 atm.) was bubbled through the mixture. Filtration through Celite and concentration of the filtrate *in vacuo* afforded **116** as a foam which solidified on standing (496 g, 98% yield).

### Example 12

**Phosphate salt of 116:** A solution of acetamido amine **116** (5.02 g, 16.1 mmol) in acetone (75 mL) at reflux was treated with 85% phosphoric acid (1.85 g, 16.1 mmol) in abs. ethanol (25 mL). Crystallization commenced immediately and after cooling to 0°C for 12 hours the precipitate was collected by filtration to afford **116•H**_{**3**}**PO**_{**4**} as long colorless needles (4.94 g, 75% yield; [α]_{D} -39.9° (c=1, water)), mp 203-4°C.

### Example 13

**Hydrochloride salt of 116:** A solution of acetamido amine 116 (2.8 g, 8.96 mmol) in abs. ethanol (9 mL) was treated with 2.08 M hydrogen chloride in ethanol (8.6 mL, 17.9 mmol). Most of the ethanol was evaporated *in vacuo* and the oily residue was stirred with ethyl acetate (20 mL) until solid formed. Hexanes (20 mL) were gradually added to the stirred mixture. After one hour at ambient temperature, the solid was collected by filtration, washed with diethyl ether and dried *in vacuo*. This afforded **116•HCl** as an off-white solid (2.54 g, 81% yield; [α]_{D} -43° (c=0.4, water)), mp206°C.

All literature and patent citations above are hereby expressly incorporated by reference in their entirety at the locations of their citation. Specifically cited sections or pages of the above cited works are incorporated by reference with specificity.

Whenever a compound described herein is substituted with more than one of the same designated group, such as, by was of example and not limitation, "R⁷", "R⁸", "R⁹", "R²⁰", or "R²²", then it will be understood that each of the groups may be the same or different, i.e., each group is independently selected. So for example, the phrase "R²² is" is synonymous with the phrase "each R²² is independently".

The invention has been described in detail sufficient to allow one of ordinary skill in the art to make and use the subject matter of the following claims. It is apparent that certain modifications of the methods and compositions of the following claims can be made within the scope and spirit of the invention.

## Claims

1. A process for the preparation of a compound of the formula: 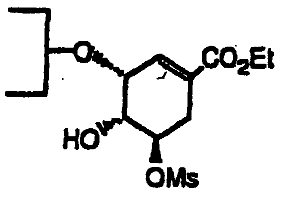 wherein Ms is mesyl;
which process comprises reaction of a compound of the formula: 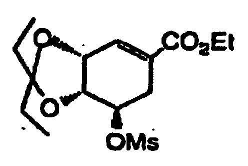 with a Lewis acid reagent.

2. A process for the preparation of a compound of the formula: 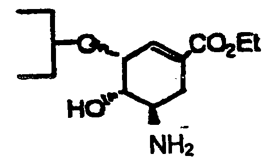 which process comprises reaction of a compound of the formula: 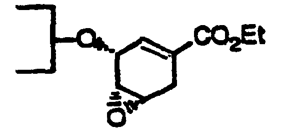 with an amine reagent.

3. A compound of the formula:

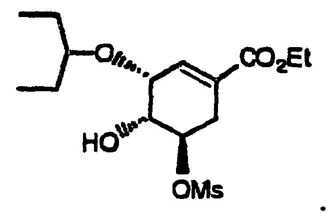

4. A compound of the formula:

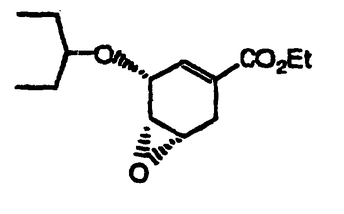

5. A compound of the formula:

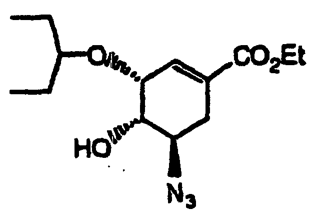

6. A compound of the formula:

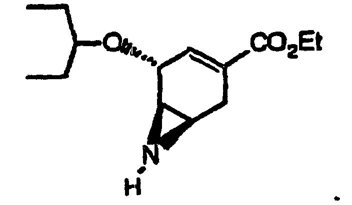

7. A compound of the formula:

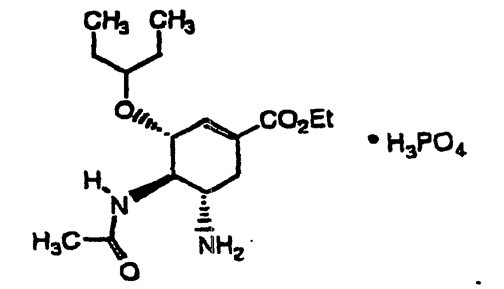

8. A process for preparing the compound of the formula 116 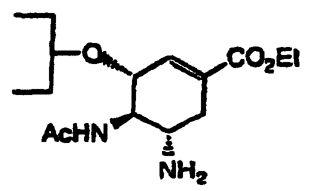 which comprises
a) converting the compound of formula 110 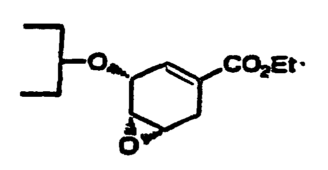 to the compound of formula 111: 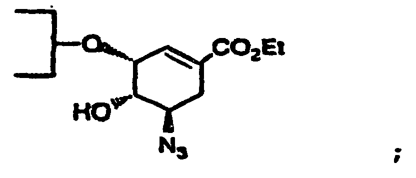
b) converting the compound of formula 111 to the compound of formula 113: 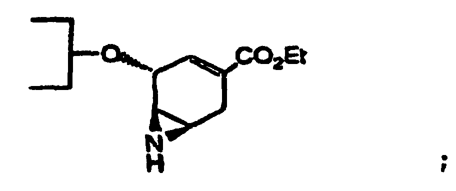
c) converting the compound of formula 113 to the compound of formula 114: 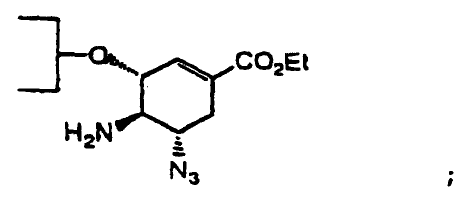
d) converting the compound of formula 114 to the compound of formula 115: 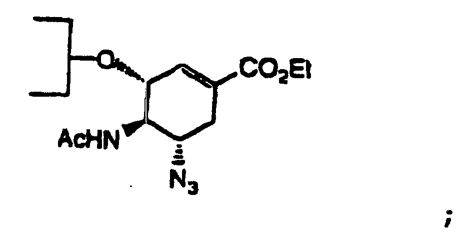 and
e) converting the compound of formula 115 to the compound of formula 116.

9. The process of claim 8 wherein
a) in step a) compound 110 is treated with sodium azide;
b) in step b) compound 111 is treated with a reducing reagent, in particular triphenylphosphine;
c) in step c) compound 113 is treated with sodium azide;
d) in step d) compound 114 is treated with an acetylating reagent; and
e) in step e) compound 115 is subjected to catalytic hydrogenation.

10. A process for preparing the compound of formula 116: 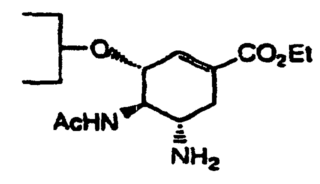 which comprises
a) converting the compound of formula 201 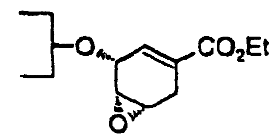 to the compound of formula 202. 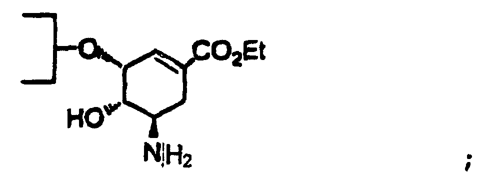
b) converting the compound of formula 202 to the compound of formula 203 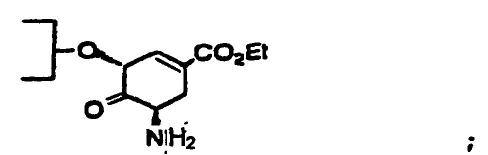
c) converting the compound of formula 203 to the compound of formula 204 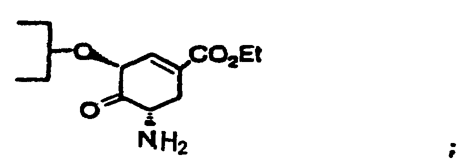
d) converting the compound of formula 204 to the compound of formula 205 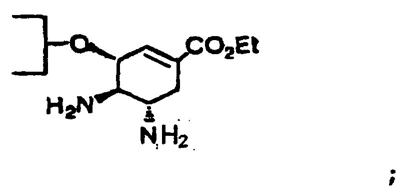 and
e) converting the compound of formula 205 to the compound of formula 116.

11. The process of claim 10 wherein
a) in step a) compound 201 is treated with an amine reagent;
b) in step b) compound 202 is treated with an oxidizing reagent;
c) in step c) compound 203 is treated with a base:
d) in step d) compound 204 is treated with a reductive amination reagent; and
e) in step e) compound 205 is treated with an acetylating reagent.

## Patentansprüche

1. Verfahren zur Herstellung einer Verbindung der Formel: 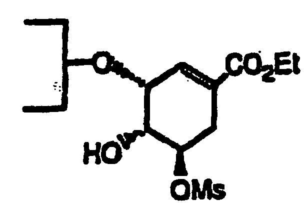 worin Ms für Mesyl steht,
bei dem man eine Verbindung der Formel 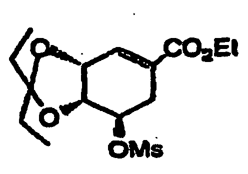 mit einem Lewis-sauren Reagenz umsetzt.

2. Verfahren zur Herstellung einer Verbindung der Formel: 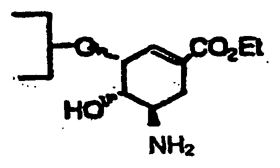 bei dem man eine Verbindung der Formel: 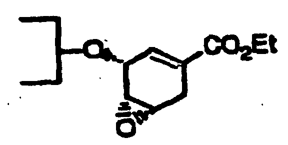 mit einem Amin-Reagenz umsetzt.

3. Verbindung der Formel:

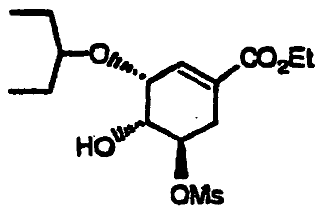

4. Verbindung der Formel:

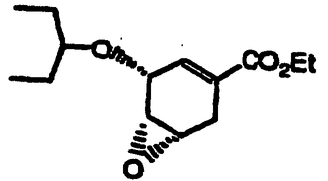

5. Verbindung der Formel:

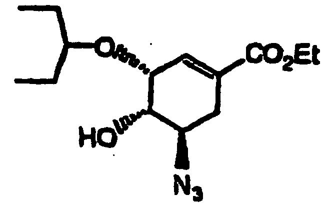

6. Verbindung der Formel:

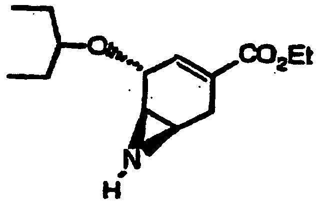

7. Verbindung der Formel:

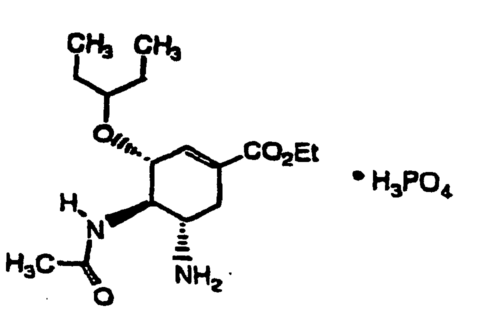

8. Verfahren zur Herstellung der Verbindung der Formel 116 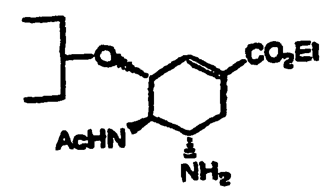 bei dem man
a) eine Verbindung der Formel 110 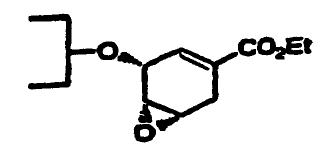 in die Verbindung der Formel 111 umwandelt: 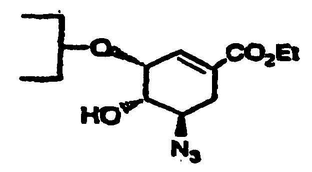
b) die Verbindung der Formel 111 in die Verbindung der Formel 113 umwandelt: 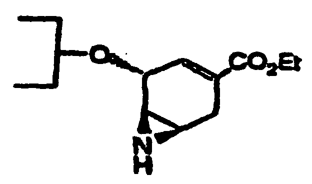
c) die Verbindung der Formel 113 in die Verbindung der Formel 114 umwandelt: 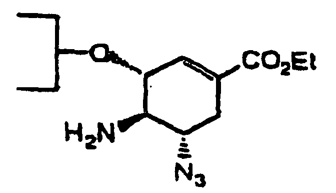
d) die Verbindung der Formel 114 in die Verbindung der Formel 115 umwandelt: 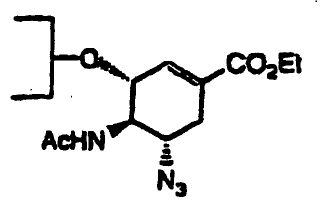 und
e) die Verbindung der Formel 115 in die Verbindung der Formel 116 umwandelt.

9. Verfahren nach Anspruch 8, bei dem
a) in Schritt a) die Verbindung 110 mit Natriumazid behandelt wird;
b) in Schritt b) die Verbindung 111 mit einem Reduktionsmittel, insbesondere Triphenylphosphin, behandelt wird;
c) in Schritt c) die Verbindung 113 mit Natriumazid behandelt wird;
d) in Schritt d) die Verbindung 114 mit einem Acetylierungsmittel behandelt wird;
e) in Schritt e) die Verbindung 115 der katalytischen Hydrierung unterworfen wird.

10. Verfahren zur Herstellung der Verbindung der Formel 116: 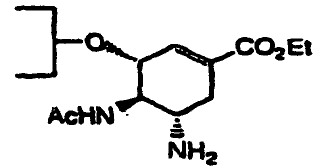 bei dem man
a) die Verbindung der Formel 201 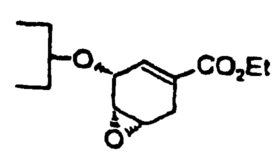 in die Verbindung der Formel 202 umwandelt: 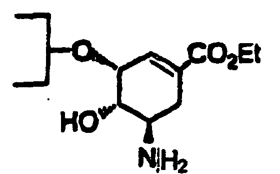
b) die Verbindung der Formel 202 in die Verbindung der Formel 203 umwandelt: 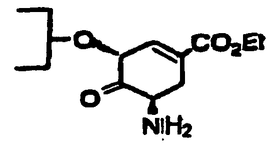
c) die Verbindung der Formel 203 in die Verbindung der Formel 204 umwandelt: 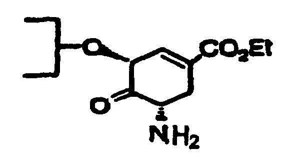
d) die Verbindung der Formel 204 in die Verbindung der Formel 205 umwandelt: 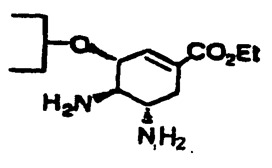 und
e) die Verbindung der Formel 205 in die Verbindung der Formel 116 umwandelt.

11. Verfahren nach Anspruch 10, bei dem man
a) in Schritt a) die Verbindung 201 mit einem Amin-Reagenz behandelt;
b) in Schritt b) die Verbindung 202 mit einem Oxidationsmittel behandelt;
c) in Schritt c) die Verbindung 203 mit einer Base behandelt;
d) in Schritt d) die Verbindung 204 mit einem reduktiyen Aminierungsmittel behandelt; und
e) in Schritt e) die Verbindung 205 mit einem Acetylierungsmittel behandelt.

## Revendications

1. Procédé pour la préparation d'un composé de la formule : 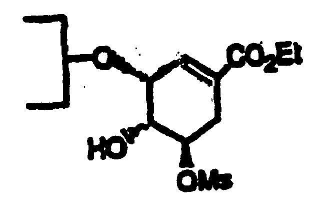 où M est mésyle ;
lequel procédé comprend la réaction d'un composé de la formule : 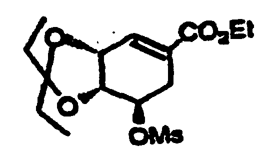 avec un réactif d'acide de Lewis.

2. Procédé pour la préparation d'un composé de la formule : 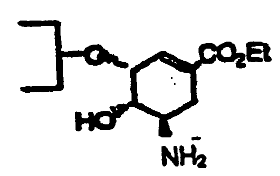 lequel procédé comprend la réaction d'un composé de la formule : 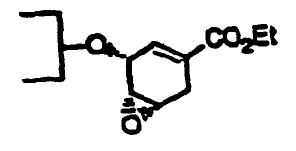 avec un réactif d'amine.

3. Composé de la formule :

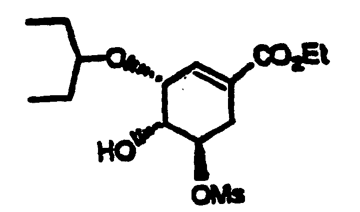

4. Composé de la formule :

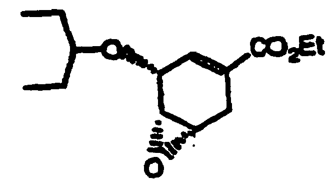

5. Composé de la formule :

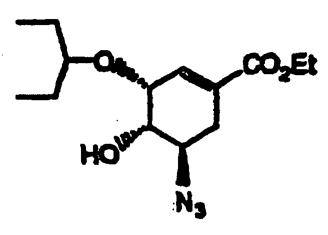

6. Composé de la formule :

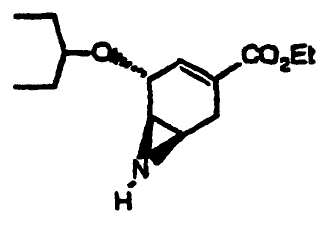

7. Composé de la formule :

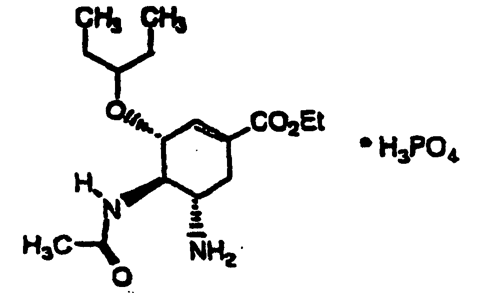

8. Procédé pour la préparation du composé de la formule 116 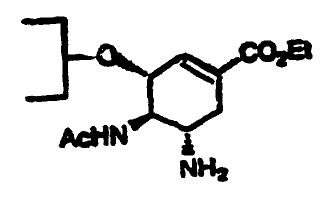 qui comprend
a) la conversion du composé de formule 110 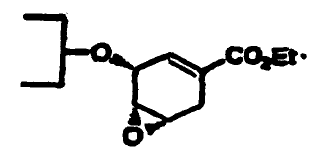 en le composé de formule 111 : 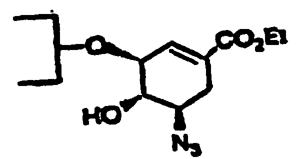
b) la conversion du composé de formule 111 en le composé de formule 113 : 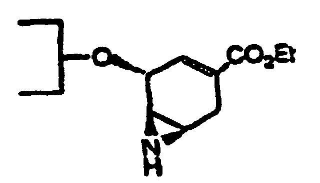
c) la conversion du composé de formule 113 en le composé de formule 114 : 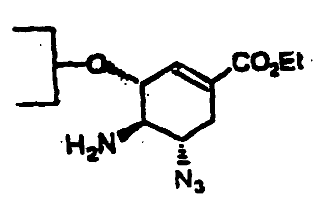
d) la conversion du composé de formule 114 en le composé de formule 115 : 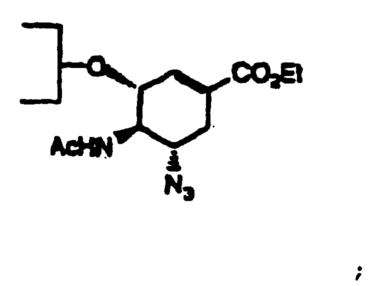 et
e) la conversion du composé de formule 115 en le composé de formule 116.

9. Procédé de la revendication 8 où
a) à l'étape a) le composé 110 est traité avec de l'azide de sodium ;
b) à l'étape b) le composé 111 est traité avec un réactif réducteur, en particulier de la triphénylphosphine ;
c) à l'étape c) le composé 113 est traité avec de l'azide de sodium ;
d) à l'étape d) le composé 114 est traité avec un réactif acétylant et
e) à l'étape e) le composé 115 est soumis à une hydrogénation catalytique.

10. Procédé de préparation du composé de formule 116 : 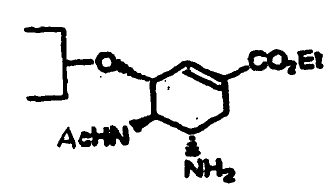 qui comprend
a) la conversion du composé de formule 201 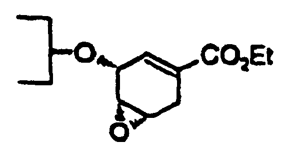 en le composé de formule 202 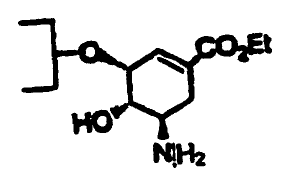
b) la conversion du composé de formule 202 en le composé de formule 203 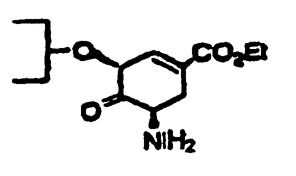
c) la conversion du composé de formule 203 en le composé de formule 204 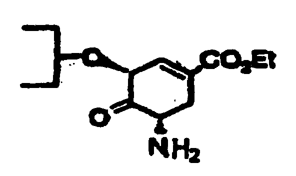
d) la conversion du composé de formule 204 en le composé de formule 205 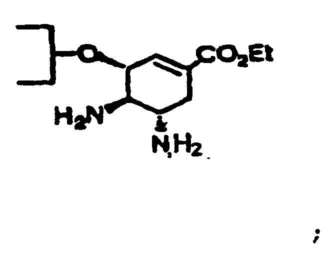 et
e) la conversion du composé de formule 205 en le composé de formule 116.

11. Procédé de la revendication 10 où
a) à l'étape a) le composé 201 est traité avec un réactif d'amine ;
b) à l'étape b) le composé 202 est traité avec un réactif oxydant ;
c) à l'étape c) le composé 203 est traité avec une base ;
d) à l'étape d) le composé 204 est traité avec un réactif d'amination réductrice ; et
e) à l'étape e) le composé 205 est traité avec un réactif acétylant.
